# EUROPEAN PATENT SPECIFICATION

(11) **EP 3 630 246 B1**
(45) Date of publication and mention of the grant of the patent: **29.03.2023**
(21) Application number: 17728287.8
(22) Date of filing: 29.05.2017
(51) Int. Cl.: A61M 16/00, A61M 16/01, A61M 16/10, A61M 16/08

(54) **ANAESTHETIC BREATHING APPARATUS**
BEATMUNGSVORRICHTUNG FÜR ANÄSTHESIE
RESPIRATEUR D'ANESTHÉSIE

(43) Date of publication of application: 08.04.2020
(73) Proprietor: Maquet Critical Care AB, 171 06 Solna (SE)
(72) Inventor: ERIKSSON, Mats, 18464 Åkersberga (SE)
(74) Representative: Zacco Sweden AB
(86) International application number: PCT/SE2017/050569
(87) International publication number: WO 2018/222092

(56) References cited:
- EP-A1- 2 474 333
- WO-A1-01/43803
- WO-A1-2010/130290
- US-A1- 2006 213 517
- US-A1- 2015 374 947

## Description

### TECHNICAL FIELD

This disclosure relates to a method for maintaining ventilation during an oxygen flush in an anaesthetic breathing apparatus. This disclosure also relates to a computer program for performing a method for maintaining ventilation during an oxygen flush in an anaesthetic breathing apparatus, and a computer program product for performing a method for maintaining ventilation during an oxygen flush in an anaesthetic breathing apparatus. This disclosure further relates to an anaesthetic breathing apparatus.

### BACKGROUND

An anaesthetic breathing apparatus may be utilised in operating rooms. An anaesthetic breathing apparatus comprises various pressure sensors and regulators, flow control devices, gas mixing devices and at least one vaporiser for vaporising a volatile liquid anaesthetic agent, abbreviated "AA", and is configured to administer gas to a patient. The gas administered to the patient comprises fresh gas, abbreviated "FG," and optionally recirculated gas from the patient. The FG may, for instance, contain AA and oxygen, O2. The patient is connected to the anaesthetic breathing apparatus via a patient interface. A breathing circuit of the anaesthetic breathing apparatus comprises an inspiratory section through which the FG is conducted to the patient interface, and an expiratory section that conveys expiratory gas from the patient interface.

During automatic ventilation of a patient, in controlled mode or support mode, the anaesthetic breathing apparatus automatically controls inspiration and expiration of the patient. A control unit of the breathing apparatus controls the fresh gas content to the patient, recirculation of expiratory gas, respiratory rate (breathing frequency), inspiration/expiration ratio, etc. During expiration, a Positive End-Expiratory Pressure, PEEP, is maintained to prevent the lungs of the patient from collapsing. In contrast, during manual ventilation, a manual bag for controlling a flow of gas into the patient is used by an operator of the anaesthetic breathing apparatus. The pressure in the breathing circuit is controlled by an Adjustable Pressure Limit valve.

In a typical anaesthetic breathing apparatus, a portion of the expiratory gas may be recirculated to the patient, i.e., the patient is connected to a substantially closed breathing circuit and rebreathing of AA is enabled via a CO2 absorber. Alternatively, the patient circuit could be an open circuit and all of the expiratory gas could simply be vented from the breathing apparatus, and not be rebreathed by the patient.

A typical function provided by an anaesthetic breathing apparatus is an oxygen flush function. The oxygen flush is manually operated by a user to provide an instant flow of O2 into the inspiratory section. The oxygen flush may be used, e.g., to rapidly supply oxygen to the patient, or to rapidly increase pressure in at least part of the inspiratory section. The oxygen flush may be activated by a user pushing a button, and may be stopped by the user releasing the button. The button may directly actuate a manual valve.

When the oxygen flush is activated, excess gas and oxygen from the flow of O2 may be released from the breathing circuit via a pressure relief valve. However, the patient may be subjected to a high flow of oxygen as well as a pressure of up to the threshold pressure of the pressure relief valve.

US 5678537 discloses a system to prevent the build-up of pressure within a patient circuit when an oxygen flush is activated in a ventilator powered anaesthesia system. In the event the user activates the oxygen flush at a time when the ventilator is in the inspiratory phase providing a breath to the patient, a valve is automatically opened to vent the patient circuit to the atmosphere, thereby preventing the additional oxygen flow resulting in an undesirable pressure build up that could adversely affect the lungs of the patient.

Such a system may reduce the risk of pressure build-up negatively affecting the lungs of a patient. However, since the patient circuit is vented to the atmosphere, the lungs of the patient may risk collapsing (i.e., atelectasis) due to loss of PEEP.

EP2474333 A1 relates to the field of mechanical ventilation for patients undergoing general anesthesia, more specifically, to a system and method for controlling the introduction of fresh gas into a breathing circuit.

### SUMMARY

It is an object according to this disclosure to overcome, or at least alleviate, at least one of the above mentioned drawbacks. To better address one or more of these concerns there are provided aspects for maintaining ventilation during an oxygen flush in an anaesthetic breathing apparatus, as defined in the independent claims below. The invention is defined by the appended claims.

According to an aspect of this disclosure, the object is achieved by a method for maintaining ventilation during an oxygen flush in an anaesthetic breathing apparatus. The anaesthetic breathing apparatus comprises a breathing circuit, a fresh gas section for providing fresh gas to a patient, and a ventilator section for providing ventilation by providing driving gas for inspiration and controlling flow of expiratory gas from the patient. The fresh gas section and the ventilator section are connected to the breathing circuit. The fresh gas section comprises an oxygen supply and at least one further gas supply, and the ventilator section comprises an expiratory pressure control valve, a driving gas line, an inspiratory pressure control valve, and a rebreathing gas reservoir. The method comprises steps of:
- starting a continuous flow of oxygen from the oxygen supply into at least a portion of the breathing circuit and interrupting an ongoing ventilation in order to activate the oxygen flush,
- maintaining the continuous flow of oxygen, and during the step of maintaining the continuous flow of oxygen preforming a step of:
- cycling the expiratory pressure control valve between an upper pressure level setting to provide an inspiratory phase of a breathing cycle and a lower pressure level setting to provide an expiratory phase of the breathing cycle.

The breathing cycles in the anaesthetic breathing apparatus are maintained during the oxygen flush by cycling the expiratory pressure control valve between an upper pressure level setting to provide an inspiratory phase of a breathing cycle and a lower pressure level setting to provide an expiratory phase of the breathing cycle during the maintained continuous flow of oxygen. Thus, a pressure build up in the lungs of the patient as well as a atelectatic collapse of the lungs of the patient are prevented. As a result, the above mentioned object is achieved.

The anaesthetic breathing apparatus may be provided for ventilating a patient. The anaesthetic breathing apparatus may comprise various sensors, regulators, flow control devices, gas mixing devices and a vaporiser for vaporising a volatile liquid anaesthetic agent, AA. The anaesthetic breathing apparatus comprises a patient interface for connecting the anaesthetic breathing apparatus to a patient. The anaesthetic breathing apparatus comprises at least one control unit and a user interface for controlling the anaesthetic breathing apparatus. The oxygen flush may be activated by an operator manipulating a dedicated push button or other type of input device of the anaesthetic breathing apparatus.

The anaesthetic breathing apparatus may be configured for automatic ventilation of a patient, in controlled mode or support mode. The patient may be ventilated with fresh gas, FG, i.e., gas from the fresh gas section containing O2, volatile anaesthetic agent AA, and optionally a further gas. The patient may also be ventilated with recirculated expiratory gas from the ventilator section. CO2 is removed from such recirculated expiratory gas. The oxygen flush function of the anaesthetic breathing apparatus interrupts the ventilation with recirculated expiratory gas and/or fresh gas, FG, instead providing a flow of pure O2 into the breathing circuit.

According to a further aspect of this disclosure, the above mentioned object is achieved by a computer program that causes an anaesthetic breathing apparatus to perform a method for maintaining ventilation during an oxygen flush in the anaesthetic breathing apparatus, wherein the computer program is embedded in a computer of the anaesthetic breathing apparatus and comprises a computer readable code configured to cause one or more calculation units of one or more control units of the anaesthetic breathing apparatus to perform the method according to any one of aspects and/or embodiments discussed herein.

According to a further aspect of this disclosure, the above mentioned object is achieved by a computer program product that is connectable to a computer of an anaesthetic breathing apparatus, wherein the computer operates the anaesthetic breathing apparatus so as to perform a method for maintaining ventilation during an oxygen flush in the anaesthetic breathing apparatus, wherein the computer program product comprises computer readable code configured to cause one or more calculation units of one or more control units of the computer of the anaesthetic breathing apparatus to perform the method according to any one of aspects and/or embodiments discussed herein.

According to a further aspect of this disclosure, the above mentioned object is achieved by an anaesthetic breathing apparatus comprising a breathing circuit, a fresh gas section for providing fresh gas to a patient, and a ventilator section for providing ventilation by providing driving gas for inspiration and controlling flow of expiratory gas from the patient. The fresh gas section and the ventilator section are connected to the breathing circuit. The fresh gas section comprises an oxygen supply and at least one further gas supply, and the ventilator section comprises an expiratory pressure control valve, a driving gas line, an inspiratory pressure control valve, and a rebreathing gas reservoir. The anaesthetic breathing apparatus is configured, upon user input, to:
- start a continuous flow of oxygen from the oxygen supply into at least a portion of the breathing circuit, in order activate an oxygen flush, and
- maintain the continuous flow of oxygen.

The anaesthetic breathing apparatus comprises one or more control units configured to:
- interrupt an ongoing ventilation, and
- cycle the expiratory pressure control valve between an upper pressure level setting to provide an inspiratory phase of a breathing cycle and a lower pressure level setting to provide an expiratory phase of the breathing cycle.

Further features of, and advantages with, the invention will become apparent when studying the appended claims and the following detailed description.

### BRIEF DESCRIPTION OF THE DRAWINGS

Various aspects and/or embodiments of the invention, including its particular features and advantages, will be readily understood from the example embodiments discussed in the following detailed description and the accompanying drawings, in which:
Fig. 1 schematically illustrates an anaesthetic breathing apparatus according to embodiments of this disclosure;
Fig. 2 schematically illustrates embodiments of a control unit of an anaesthetic breathing apparatus;
Fig. 3 illustrates embodiments of a method for maintaining ventilation during an oxygen flush in an anaesthetic breathing apparatus;
Figs. 4a - 4d illustrate volume flow to a patient, patient pressure, and expiratory pressure control valve pressure level during various modes of operation during ventilation of a patient; and
Fig. 5 illustrates a computer program product according to embodiment of this disclosure.

### DETAILED DESCRIPTION

Aspects and/or embodiments of this disclosure will now be described more fully. Like numbers refer to like elements throughout. Well-known functions or constructions will not necessarily be described in detail for brevity and/or clarity.

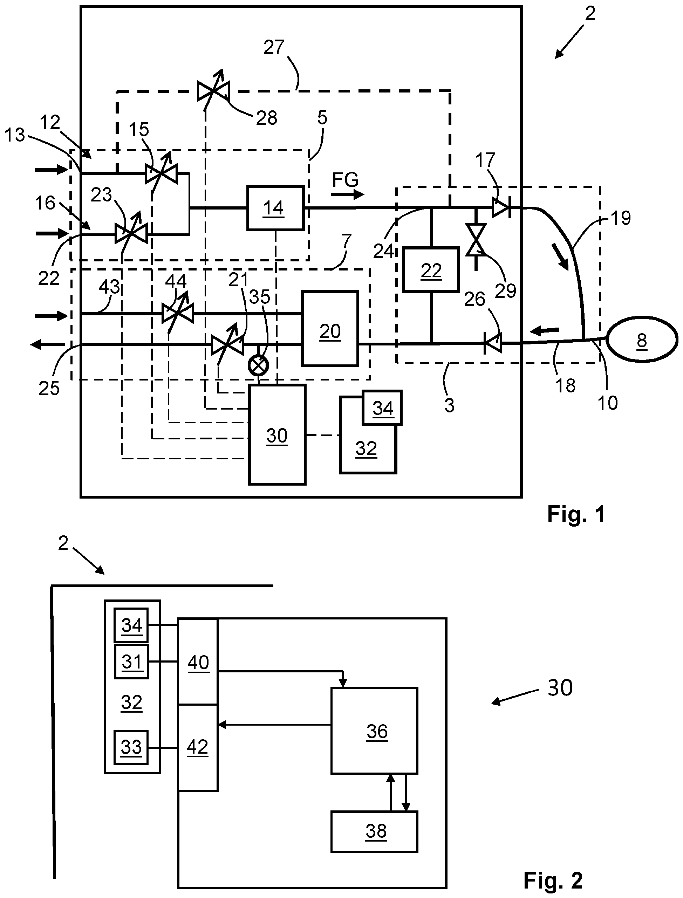

Fig. 1 schematically illustrates an anaesthetic breathing apparatus 2 according to embodiments of this disclosure. The breathing apparatus 2 comprises a breathing circuit 3, a fresh gas section 5, and a ventilator section 7. The fresh gas section 5 is configured for providing fresh gas, FG, to a patient 8. The ventilator section 7 is configured for conducting expiratory gas from the breathing apparatus 2 and for returning recirculated expiratory gas to the breathing circuit. A patient interface 10 connects the breathing circuit 3 to the patient 8. The patient interface 10 may be any suitable patient interface, such as a face mask, tracheal tube, or larynx mask.

The fresh gas section 5 comprises an oxygen supply 12 and at least one further gas supply 16. The oxygen supply 12 of the breathing apparatus 2 comprises at least an O2 gas inlet 13. The oxygen supply 12 may further comprise an oxygen valve 15. According to some embodiments, the oxygen supply 12 may also comprise an O2 gas source. Similarly, the at least one further gas supply 16 may comprise at least a further gas inlet 22. The at least one further gas supply 16 may further comprise a further gas control valve 23. According to some embodiments, the at least one further gas supply 16 may comprise a source of further gas.

The further gas may, for instance, be air and/or nitrous oxide. Seen in a direction of gas flow towards the patient 8, the fresh gas section 5 further comprises an anaesthetic agent vaporizer 14. Conduits of the fresh gas section 5 fluidly connect the different components of the fresh gas section 5. The fresh gas section 5 is fluidly connected to the breathing circuit 3.

The breathing circuit 3 comprises a first check valve 17. A patient inspiration tube 19 connected to the breathing apparatus 2 connects the breathing circuit 3 to the patient interface 10. The breathing circuit 3 further comprises a second check valve 26. A patient expiration tube 18 connected to the breathing apparatus 2 connects the patient interface 10 to the breathing circuit 3. Also, a CO2 absorber 22 is arranged in the breathing circuit 3, between the first and second check valves 17, 26. The CO2 absorber 22 absorbs CO2 from expiratory gas recirculated from the ventilator section 7 to the breathing circuit 3 during an inspiratory phase. The CO2 absorber 22 is fluidly connected at a recirculation connection point 24 downstream of the fresh gas section 5.

The ventilator section 7 comprises an expiratory pressure control valve, 21, which sometimes may be referred to as a PEEP valve 21, a driving gas line 43 for providing a driving gas, an inspiratory pressure control valve 44, and a rebreathing gas reservoir 20. The ventilator section 7 leads expiratory gas from the patient 8 to an exhaust port 25 of the breathing apparatus 2. The rebreathing gas reservoir 20, such as e.g. a volume reflector (known e.g. from EP 2429622), a carbon reflector, bag in bottle (known e.g. from US 5678537), or similar device, is arranged in the ventilator section 7, between the patient expiration tube 18 and the expiratory pressure control valve 21. It may be noted that, if a carbon reflector is used the reservoir is not a volume containing the expired aesthetic gas, but a reservoir containing aesthetic agent adsorbed during last expiration, said AA being desorbed by the supplied driving gas during next inspiration phase, see e.g. US 5471979 A1. The driving gas line 43 and the inspiratory pressure control valve 44 are connected to the rebreathing gas reservoir 20. Suitable non-limiting examples of a driving gas are oxygen or air. Conduits of the ventilator section 7 connect the different components of the ventilator section 7. The breathing circuit 3 is fluidly connected with the ventilator section 7.

Thus, using the rebreathing gas reservoir 20, expiratory gas containing anaesthetic agent and cleaned of CO2 by the CO2 absorber 22 may be recirculated to the patient 8 in a known manner. Briefly, during the expiratory phase, the first check valve 17 prevents expiratory gas from the patient 8 to flow towards the fresh gas section 5. Instead expiratory gas flows through the second check valve 26 and fills the rebreathing gas reservoir 20. The expiratory pressure control valve 21 controls the pressure against which the patient exhales. The inspiratory pressure control valve 44 is closed during the expiratory phase. During the inspiratory phase, the expiratory pressure control valve 21 is closed and the inspiratory pressure control valve 44 is opened, pushing the expiratory gas from the rebreathing gas reservoir 20 back into the breathing circuit 3. The second check valve 26 prevents the expiratory gas from flowing directly back to the patient 8. Instead the gas flows through the CO2 absorber 22 and to the patient 8, via the first check valve 17. The rebreathed expiratory gas is complemented with FG, containing O2, AA, and optionally further gas, from the fresh gas section 5 to appropriate O2 and AA levels for the patient 8. FG may be provided at a low constant flow or alternatively, may be shut off during the expiratory phase and turned on during the inspiratory phase.

Alternatively, the breathing circuit may be an open circuit and all of the expiratory gas could simply be vented from the breathing apparatus, and not rebreathed by the patient. In such a breathing apparatus, neither the rebreathing gas reservoir 20 nor the CO2 absorber 22 are required.

The breathing apparatus 2 comprises a pressure relief valve 29. The pressure relief valve 29 releases gas from the breathing apparatus 2 in case a gas pressure in the breathing circuit 3 exceeds a threshold pressure level.

The breathing apparatus 2 comprises an oxygen flush section that provides an oxygen flush function. For this purpose, the breathing apparatus 2 may comprise a dedicated O2 conduit 27 comprising a dedicated oxygen flush valve 28 and leading from the O2 gas inlet 13 to the breathing circuit 3 upstream of the first check valve 17, or to the fresh gas section 5 downstream of the anaesthetic agent vaporiser 14. Alternatively, the dedicated O2 conduit 27 may be omitted and the oxygen valve 15 may be controlled to provide an oxygen flush. In such case the anaesthetic agent vaporiser 14 has to be switched off, or bypassed. Other ways of providing an oxygen flush may also be utilised. This technical solution, as discussed herein, is not limited to any particular hardware solution for providing an oxygen flush, but is based around a maintained ventilation of the patient 8 during an oxygen flush when only O2 is flowing into the breathing circuit 3.

The anaesthetic breathing apparatus 2 comprises one or more control units 30 configured to control the operation of the breathing apparatus 2. Thus, the one or more control units 30 may control at least some of the controllable valves of the breathing apparatus 2 and the vaporiser 14. Sensors may be connected to the one or more control units 30, such as a pressure sensor 35 for sensing a pressure in the ventilator section 7 upstream of the expiratory pressure control valve 21. The one or more control units 30 may control ventilation of the patient 8 in one or more different ventilation modes, such as pressure controlled ventilation, pressure support ventilation, volume controlled ventilation, volume support ventilation, and/or other modes of ventilation. During ventilation of the patient 8, the control unit 30 controls the breathing apparatus 2 to provide a series of breathing cycles, each breathing cycle comprising an inspiratory phase and an expiratory phase. During the inspiratory phase, the driving gas pushes expiratory gas from the rebreathing gas reservoir 20 via the CO2 absorber 22 to the patient 8, and FG is supplied from fresh gas section 5. During the expiratory phase, expiratory gas is conducted from the patient 8 into the ventilator section 7 and the rebreathing gas reservoir 20. Excess expiratory gas is conducted towards the outlet port 25.

An operator may provide input to the breathing apparatus 2 via a user interface 32 connected to the one or more control units 30. The user interface 32 may, for example, comprise input devices such as in the form of one or more push buttons, dials, and/or touch screens. In particular, the user interface 32 may comprise a dedicated input device 34 for controlling an oxygen flush in the breathing apparatus 2. The dedicated input device 34 may be configured for electronic control of the dedicated oxygen flush valve 28, or the oxygen valve 15, as indicated in **Fig. 1****.** Alternatively, the dedicated input device 34 may be mechanically connected to the dedicated oxygen flush valve 28, or the oxygen valve 15. The breathing apparatus 2 may provide output to an operator of the breathing apparatus 2. Thus, the user interface 32 may comprise output devices such as one or more lamps, sound emitting devices, and/or screens.

**Fig. 2** schematically illustrates embodiments of a control unit 30 of an anaesthetic breathing apparatus 2, such as the anaesthetic breathing apparatus 2 of **Fig. 1****.** The control unit 30 comprises a calculation unit 36 which may take the form of substantially any suitable type of processor circuit or microcomputer, e.g. a circuit for digital signal processing (digital signal processor, DSP), a Central Processing Unit (CPU), a processing unit, a processing circuit, a processor, an Application Specific Integrated Circuit (ASIC), a microprocessor, or other processing logic that may interpret and execute instructions. The herein utilised expression "calculation unit" may represent a processing circuitry comprising a plurality of processing circuits, such as, for example, any, some or all of the ones mentioned above. The control unit 30 comprises a memory unit 38. The calculation unit 36 is connected to the memory unit 38, which provides the calculation unit 36 with, for example, stored programme code and/or stored data which the calculation unit 36 needs to enable it to do calculations. The calculation unit 36 is also adapted to storing partial or final results of calculations in the memory unit 38. The memory unit 38 may comprise a physical device utilised to store data or programs, i.e., sequences of instructions, on a temporary or permanent basis. According to some embodiments, the memory unit 38 may comprise integrated circuits comprising silicon-based transistors. The memory unit 38 may comprise, e.g., a memory card, a flash memory, a USB memory, a hard disc, or another similar volatile or non-volatile storage unit for storing data such as e.g. ROM (Read-Only Memory), PROM (Programmable Read-Only Memory), EPROM (Erasable PROM), EEPROM (Electrically Erasable PROM), etc. in different embodiments.

The control unit 30 is further provided with respective devices 40, 42 for receiving and/or sending input and output signals. These input and output signals may comprise waveforms, pulses or other attributes which the input signal receiving devices 40 can detect as information and which can be converted to signals processable by the calculation unit 36. These signals are then supplied to the calculation unit 36. The output signal sending devices 42 are arranged to convert calculation results from the calculation unit 36 to output signals for conveying to components of the breathing apparatus 2. The input signal receiving devices 40 are connected to the user interface 32, and in particular, input devices 31 of the user interface 32, such as the above exemplified push buttons, dials, and/or touch screens. The input signal receiving devices 40 may further be connected to sensors, such as, e.g., the pressure sensor 35, of the breathing apparatus 2. The output signal sending devices 42 are connected to the user interface 32 and, in particular, to output devices 33 of the user interface 32, such as the above exemplified lamps, sound emitting devices, and/or screens. The output signal sending devices 42 may further be connected to the controllable valves 15, 23, 21 and the vaporiser 14 of the breathing apparatus 2. Each of the connections to the devices 40, 42, for receiving and sending input and output signals respectively, may take the form of one or more forms selected from among a cable, a data bus, or a wireless connection.

Referring to **Figs. 1 and 2****,** in order to provide an oxygen flush, the one or more control units 30 are configured to:
- Start a continuous flow of oxygen from the oxygen supply 12 into at least a portion of the breathing circuit 3 interrupting an ongoing ventilation of the patient 8, in order to activate an oxygen flush in the breathing apparatus 2.
- Maintain the continuous flow of oxygen from the oxygen supply 12 through these structures.
- Cycle the expiratory pressure control valve 21 between an upper pressure level setting to provide an inspiratory phase of a breathing cycle and a lower pressure level setting to provide an expiratory phase of the breathing cycle.

The continuous flow of oxygen may be started by an operator of the breathing apparatus 2 manipulating the dedicated input device 34 of the user interface 32. In response thereto, the control unit 30 controls, for example, the dedicated oxygen flush valve 28 of the dedicated O2 conduit 27 to provide a continuous flow of oxygen from the oxygen supply 12 into the breathing circuit 3, when the breathing apparatus 2 is one that comprises a dedicated O2 conduit 27. The continuous flow of oxygen into the breathing circuit 3 may be provided into any suitable position of the breathing circuit 3 or, alternatively, the continuous flow of oxygen may be provided into a different portion of the breathing apparatus 2, which is configured to conduct the oxygen flow towards the patient 8. If the breathing apparatus 2 does not comprise any dedicated O2 conduit 27, then in such embodiments the control unit 30 may be configured to control the oxygen valve 15 to provide a continuous flow of oxygen from the oxygen supply 12 into the breathing circuit 3 in response to the operator manipulating the dedicated input device 34. Accordingly, the relevant valve 28, 15 for providing the oxygen flush is electronically controlled depending upon the embodiment of the breathing apparatus 2 (i.e., whether provided with a dedicated O2 conduit 27 or not).

The term "ongoing ventilation", which is interrupted as the continuous flow of oxygen is started, relates to an ordinary ventilation operation of the anaesthetic breathing apparatus, wherein the patient is ventilated with FG, and/or recirculated expiratory gas.

The continuous flow of oxygen may be maintained by the control unit 30 maintaining the relevant valve 28, 15 at least partially open.

According to alternative embodiments wherein the breathing apparatus 2 comprises a dedicated input device, which is mechanically linked to the dedicated oxygen flush valve 28, or to the oxygen valve 15, the anaesthetic breathing apparatus 2 is configured, upon user input, i.e., a user manipulating the dedicated input device, to:
- Start a continuous flow of oxygen from the oxygen supply into at least a portion of the breathing circuit 3 in order to activate an oxygen flush, and
- maintain the continuous flow of oxygen from the oxygen supply through these structures.

In order to control a breathing cycle during the oxygen flush, the one or more control units 30 are configured to:
- Interrupt an ongoing ventilation, and
- cycle the expiratory pressure control valve 21 between an upper pressure level setting to provide an inspiratory phase of a breathing cycle and a lower pressure level setting to provide an expiratory phase of the breathing cycle.

The continuous flow of oxygen may be started by an operator of the breathing apparatus 2 manipulating the dedicated input device 34. For instance, the dedicated oxygen flush valve 28 of the dedicated O2 conduit 27 provides a continuous flow of oxygen from the oxygen supply 12 into the breathing circuit 3, if the breathing apparatus 2 comprises a dedicated O2 conduit 27. The continuous flow of oxygen into the breathing circuit 3 may be provided into any suitable position of the breathing circuit 3 alternatively, the continuous flow of oxygen may be provided into a different portion of the breathing apparatus 2, which is configured to conduct the oxygen flow towards the patient 8. If the breathing apparatus 2 does not comprise any dedicated O2 conduit 27, the oxygen valve 15 provides a continuous flow of oxygen from the oxygen supply 12 into the breathing circuit 3 in response to the operator manipulating the dedicated input device 34.

In both embodiments, i.e., comprising electronically or mechanically controlled valves 28, 15 for oxygen flush, the control unit 30 is configured to control the expiratory pressure control valve 21 to be cycled between the two pressure level settings providing an expiratory phase and an inspiratory phase of a breathing cycle. Thus, a number of consecutive breathing cycles may be provided by the anaesthetic breathing apparatus 2 to the patient 8 during the oxygen flush and the patient 8 is ventilated with oxygen, O2, provided to the breathing circuit during the oxygen flush.

The continuous flow of oxygen during the oxygen flush will cycle between being provided to the patient 8 and bypassing the patient 8. When the expiratory pressure control valve 21 is set at the upper pressure level setting, the continuous flow of oxygen will be led to the patient 8, providing the inspiratory phase of a breath with pure oxygen or at least very high levels of oxygen consistent with the oxygen flush. When the expiratory pressure control valve 21 is set at the lower pressure level setting, the expiratory phase of the breath is provided, and the continuous flow of oxygen bypasses the patient 8 by traveling from the patient inspiration tube 19 directly to the patient expiration tube 18, and then the continuous flow of oxygen continues via the ventilator section 7 to the outlet port 25.

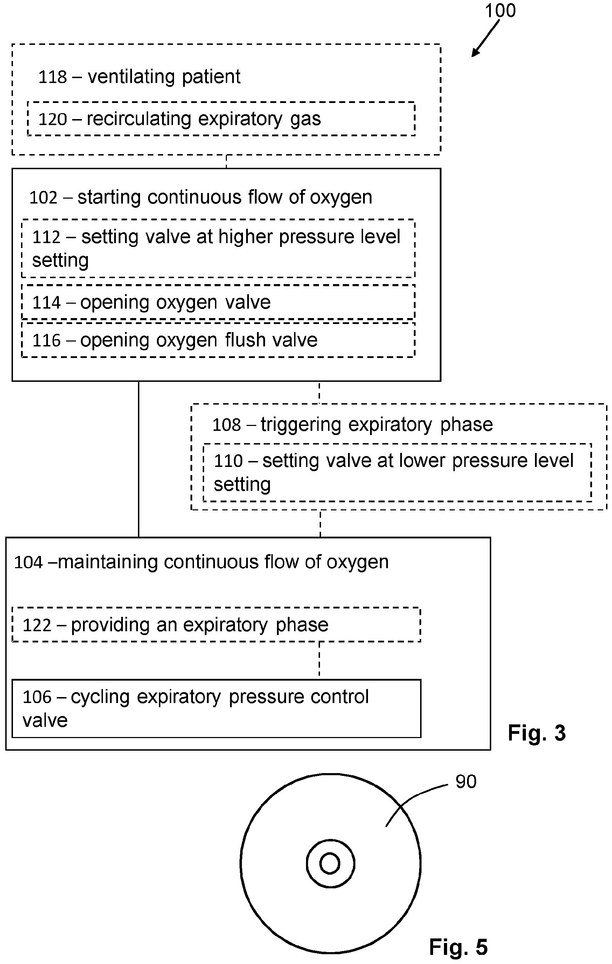

The lower pressure level setting is a pressure level setting of the expiratory pressure control valve 21 that is lower than the upper pressure level setting. The lower pressure level setting may be either a fixed lower pressure level setting during the expiratory phase, or a varying lower pressure level setting during the expiratory phase. In the latter case, mentioned purely as an example, the expiratory pressure control valve 21 may be controlled to a pressure level setting corresponding substantially to ambient pressure, i.e. corresponding to a substantially fully opened expiratory pressure control valve 21, during an initial part of the expiratory phase to minimise the expiratory resistance a patient experiences. Thereafter, the lower pressure level setting may be raised during a final part of the expiratory phase to provide an expiratory resistance preventing the lungs of the patient from collapsing. Varying pressure levels of an expiratory pressure control valve during the expiratory phase are discussed in connection with ordinary ventilation in US 6564798 B1 and US 6192885 B1, and may be at least partially utilised in some embodiments of the method for maintaining ventilation during an oxygen flush in an anaesthetic breathing apparatus discussed herein. Similarly, the upper pressure level setting may be either a fixed upper pressure level setting during the inspiratory phase, or a varying upper pressure level setting during the inspiratory phase. The pressure sensor 35 may be utilised for controlling the expiratory pressure control valve 21 between the upper and lower pressure level settings. Example levels of the lower and upper pressure level settings are discussed further below with reference to **Fig. 3** and the method 100.

The oxygen flush and thus, the cycling of the expiratory pressure control valve 21, may be interrupted when the operator of the breathing apparatus 2 stops manipulating the dedicated input device 34, or when the operator again manipulates the dedicated input device 34, or when the operator manipulates a further input device for stopping the oxygen flush. For instance, in embodiments wherein the input device is a button actuator, the oxygen flush may start when the operator of the breathing apparatus 2 pushes the button actuator, and the oxygen flush may stop when the operator releases the input device 34.

Upon interruption of the oxygen flush, the expiratory pressure control valve 21 may be set at the lower pressure level setting to trigger an expiratory phase in order to terminate a current breathing cycle by emptying the lungs of the patient 8. Thereafter, ordinary ventilation of the patient 8 with FG, and/or recirculated expiratory gas, starts again.

The at least one control unit 30 is configured to control the breathing apparatus 2 and relevant components thereof, such as a valve 28, 15 which controls oxygen flow, and the expiratory pressure control valve 21, to perform steps of the method 100 for maintaining ventilation during an oxygen flush in an anaesthetic breathing apparatus discussed herein.

Thus, according to a further aspect, the disclosure also relates to a method 100 for maintaining ventilation during an oxygen flush in an anaesthetic breathing apparatus 2.

**Fig. 3** illustrates embodiments of such a method 100. In the following, reference is also made to **Figs. 1 and 2****.**

The method 100 for maintaining ventilation during an oxygen flush in an anaesthetic breathing apparatus 2 may be performed in a breathing apparatus 2 as disclosed in **Fig.1****,** but is not limited to be performed in the disclosed breathing apparatus 2. However, the anaesthetic breathing apparatus 2 comprises a breathing circuit 3, a fresh gas section 5, and a ventilator section 7. The fresh gas section 5 comprises an oxygen supply 12 and at least one further gas supply 16. The ventilator section 7 comprises an expiratory pressure control valve, PEEP valve 21, for controlling expiratory pressure.

The method 100 comprises steps of:
- starting 102 a continuous flow of oxygen from the oxygen supply 12 into at least a portion of the breathing circuit 3 interrupting an ongoing ventilation in order to activate the oxygen flush,
- maintaining 104 the continuous flow of oxygen, and during the step of maintaining 104 the continuous flow of oxygen preforming a step of:
- cycling 106 the expiratory pressure control valve 21 between an upper pressure level setting to provide an inspiratory phase of a breathing cycle and a lower pressure level setting to provide an expiratory phase of the breathing cycle.

The method 100 provides an oxygen flush in the breathing apparatus 2. The step of starting 102 a continuous flow of oxygen into at least a portion of the breathing circuit 3, means that the anaesthetic breathing apparatus 2, during ventilation of the patient 8 with fresh gas and recirculated expiratory gas, is interrupted in order to perform at the least the above mentioned method steps 104 and 106. Thus, the step of starting 102 a continuous flow of oxygen, interrupts the breathing gas flow from the ventilator section 7 and the fresh gas section 5. The pressure in the breathing circuit 3 is maintained by the steps of maintaining 104 the continuous flow of oxygen, and of cycling 106 the expiratory pressure control valve 21.

In embodiments comprising an electronically controlled dedicated oxygen flush valve 28, or oxygen control valve 15, the step of starting 102 a continuous flow of oxygen from the oxygen supply may comprise the control unit 30 of the anaesthetic breathing apparatus 2 starting the continuous flow of oxygen upon receiving an initiation signal, from an operator of the anaesthetic breathing apparatus. The initiation signal may be provided by the dedicated input device 34 upon manipulation thereof by the operator.

According to one set of embodiments of this disclosure, the fresh gas section 5 comprises an oxygen valve 15 for controlling flow of oxygen, and the step of starting 102 a continuous flow of oxygen from the oxygen supply 12 may comprise a step of:
- opening 114 at least partially the oxygen valve 15. In this manner, the oxygen valve 15 of the oxygen supply 12 may be utilised for performing the oxygen flush. Thus, no dedicated O2 conduit may be required in the breathing apparatus 2. During the oxygen flush, the vaporiser 14 is deactivated, i.e., is not providing any vaporised anaesthetic agent to the patient 8. This, may be achieved, for example, with a bypass conduit inside the vaporiser 14, or with a bypass conduit around the vaporiser 14. Thus, during the oxygen flush, the oxygen bypasses anaesthetic agent vaporising components of the vaporiser 14.

According to embodiments of this disclosure, the anaesthetic breathing apparatus 2 may comprise a dedicated oxygen flush valve 28 connected to the oxygen supply 12, and the step of starting 102 a continuous flow of oxygen from the oxygen supply 12 may comprise a step of:
- opening 116 at least partially the dedicated oxygen flush valve 28. In this manner, the dedicated oxygen flush valve 28 may be utilised for performing the oxygen flush. Thus, via the dedicated O2 conduit 27, oxygen may be provided to the breathing circuit 3 and to the patient 8 upon starting of the oxygen flush.

The step of maintaining 104 the continuous flow of oxygen may be performed by maintaining the dedicated oxygen flush valve 28, or the oxygen control valve 15, open as explained above with reference to **Figs. 1 and 2****.**

The step of cycling 106 between the lower and upper pressure level settings ensures that a pressure build up in the lungs of the patient 8 and an atelectatic collapse of the lungs of the patient 8 is prevented. In essence, ventilation of the patient 8 is continued with the oxygen, O2, provided to the breathing circuit during the O2 flush, replacing the aesthetic gas mixture in the breathing circuit during the oxygen flush.

According to embodiments of this disclosure, the step of starting 102 a continuous flow of oxygen may be preceded by a step of:
- ventilating 118 a patient 8 in a volume or pressure controlled mode of ventilation of the anaesthetic breathing apparatus 2, or in a volume or pressure support mode of ventilation of the anaesthetic breathing apparatus 2. In this manner, the patient 8 may be ventilated in one or more well defined modes of ventilation prior to starting of the oxygen flush.

The volume and pressure controlled modes, as well as the volume and pressure support modes, are well known in the art of ventilation. Briefly, in controlled modes of ventilation the breathing apparatus 2 ventilates a patient at a predefined respiratory rate (breathing frequency), either providing a predefined volume of gas to the patient, or building up a predefined gas pressure to the patient, or providing gas to the patient in accordance with a predefined gas pressure profile. In support modes of ventilation, instead of using a predefined respiratory rate, each breath is initiated by detecting spontaneous breathing efforts of the patient, which then is supported by the breathing apparatus providing either a predefined volume of gas, or building up a predefined gas pressure to the patient, or providing gas to the patient in accordance with a predefined gas pressure profile.

In all modes of ventilation, recirculated expiratory gas may be returned to the breathing circuit 3. Thus, according to embodiments of this disclosure, the step of ventilating 118 a patient 8 may comprise a step of:
- recirculating 120 a portion of expiratory gas from the patient 8 in the anaesthetic breathing apparatus 2. In this manner, expiratory gas from the patient 8 still containing anaesthetic agent may be reused. The recirculated expiratory gas is cleaned of CO2 while in the CO2 absorber 22.

During an oxygen flush according to the method 100, the step of cycling 106 may be performed in a pressure controlled mode of ventilation. If the ventilation of a patient prior to performing an oxygen flush, according to the method 100, is performed in a volume controlled mode or volume support mode, this mode of ventilation is temporarily deactivated and may be resumed once the oxygen flush is stopped.

According to embodiments of this disclosure, the step of cycling 106 the expiratory pressure control valve 21 between the lower pressure level setting and the upper pressure level setting may be performed at a previously set respiratory rate and a previously set ratio between the inspiratory phase and the expiratory phase, I:E ratio. In this manner, the ventilation during the oxygen flush may be performed with the same respiratory rate and I:E ratio, for example, as used during ordinary ventilation with the breathing apparatus 2, as for instance in the step of ventilating 118, or according to a pre-programmed respiratory rate and I:E ratio for use specifically during oxygen flush. Such pre-programmed rates and ratios may be set by an operator of the breathing apparatus 2, or may be provided in software supplied, for example, by a manufacturer of the breathing apparatus 2.

Similarly, the lower and upper pressure level settings may be set by an operator of the breathing apparatus 2, or may be provided in software supplied, for example, by a manufacturer of the breathing apparatus 2. Alternatively, the breathing apparatus 2 may adapt the lower and upper pressure level settings to the relevant patient 8 being ventilated.

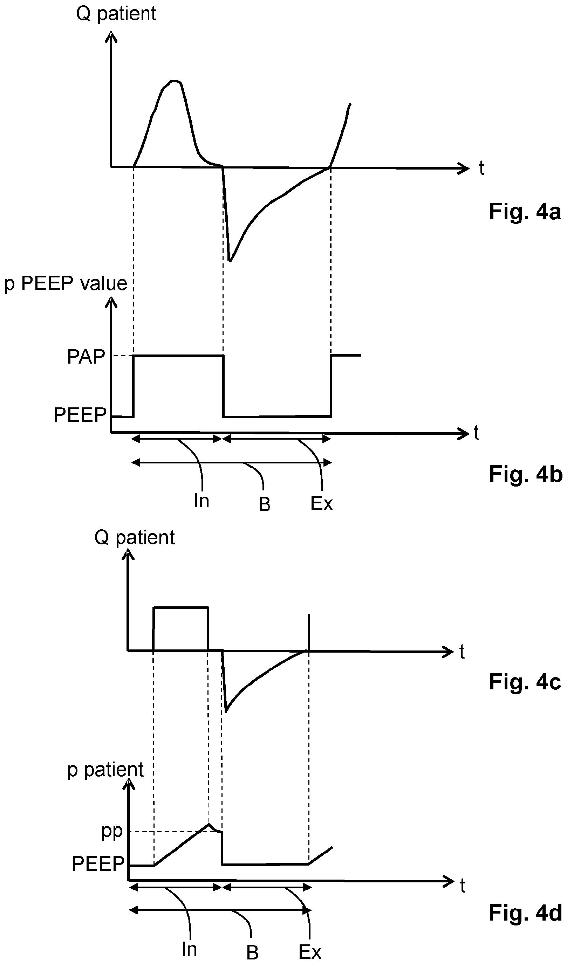

According to embodiments of this disclosure, the lower pressure level setting may be a PEEP level setting of the expiratory pressure control valve 21. In this manner, a suitable lower pressure level for preventing atelectatic collapse of the lungs of the patient 8 may be provided. The PEEP level, Positive End-Expiratory Pressure level, is the positive pressure within the lungs at the end of the expiratory phase of a breath of the patient 8, which is a well-defined pressure level of the breathing apparatus 2. The PEEP level is a key parameter set by an operator of the breathing apparatus 2 to prevent the lungs of the patient from collapse due to atelectasis. The PEEP level during the oxygen flush may be the same as the level set by the operator for the breathing mode performed prior to the oxygen flush, or a factory setting of the breathing apparatus 2. See also **Figs. 4a** - **4d** below.

According to embodiments of this disclosure, the upper pressure level setting may be at a level below a threshold pressure level of a pressure relief valve 29 of the anaesthetic breathing apparatus 2. In this manner, pressure relief via the pressure relief valve 29 may be avoided during performing of the method 100 discussed herein. Thus, ventilation during an oxygen flush may be performed.

According to embodiments of this disclosure, the upper pressure level setting may be at a plateau pressure level as determined at inspiratory zero-flow to a patient 8. In this manner, an upper pressure level adapted to the relevant patient 8 may be provided. During a volume controlled or support mode of ventilation, e.g., as performed according to the step of ventilating 118, the breathing apparatus 2 may determine the plateau pressure level of the relevant patient 8 and store the plateau pressure level in the memory unit 38 of the control unit 30. See also **Figs. 4a** - **4d** below.

According to embodiments of this disclosure, the upper pressure level may be at a Pressure Above PEEP level (abbreviated "PAP" level). In this manner, an upper pressure level utilised during pressure controlled or support mode of ventilation may be utilised as the upper pressure level during the oxygen flush. The PAP level during the oxygen flush may be the same as the PAP level set by an operator of the breathing apparatus 2 for the breathing mode performed prior to the oxygen flush, or a factory setting of the breathing apparatus 2. See also **Figs. 4a** - **4d** below.

The method 100 may be initiated at any time during ordinary ventilation of the patient 8, i.e., during the step of ventilating 118. Accordingly, the breathing apparatus 2 is configured to handle situations when the method 100 is not initiated exactly in between two breaths.

According to embodiments of this disclosure, when the step of starting 102 a continuous flow of oxygen is performed, the method 100 may comprise a substantially simultaneous step of:
- triggering 108 an expiratory phase with the expiratory pressure control valve 21 by performing a step of:
- setting 110 the expiratory pressure control valve 21 at the lower pressure level setting. In this manner, an expiratory phase may be triggered as the oxygen flush is initiated in order to terminate a current breathing cycle by emptying the lungs of the patient 8, and thereafter to ventilate during the oxygen flush by performing the step of cycling 106 the expiratory pressure control valve 21.

According to embodiments of this disclosure, when the step of starting 102 a continuous flow of oxygen is performed amidst an inspiratory phase during ventilation, the inspiratory phase is continued by performing a step of:
- setting 112 the expiratory pressure control valve 21 at the higher pressure level setting for a remaining time period of the inspiratory phase, and the step of cycling 106 the expiratory pressure control valve 21 is preceded by a step of:
- providing 122 an expiratory phase supplementing the inspiratory phase to complete a breathing cycle by setting the expiratory pressure control valve 21 at the lower pressure level setting. In this manner, an ongoing inspiratory phase may be continued as the oxygen flush is initiated in order to thereafter ventilate during the oxygen flush by performing the step of cycling 106 the expiratory pressure control valve 21; however, starting with the pressure control valve 21 at the lower pressure level provides an expiratory phase supplementing the inspiratory phase to complete a breathing cycle.

**Figs. 4a** - **4d** illustrate volume flow to a patient (Q patient), patient pressure (p patient), and expiratory pressure control valve pressure level (p PEEP value) during various modes of operation during ventilation of a patient. That is, these figures provide a graphical representation with respect to certain relationships during the above discussed step of ventilating 118 of a patient 8. The **Figs. 4a** - **4d** are used for inter alia showing some of the pressure level settings to be used in the above discussed step of cycling 106 the expiratory pressure control valve 21 between an upper pressure level setting to provide an inspiratory phase of a breathing cycle and a lower pressure level setting to provide an expiratory phase of the breathing cycle. A breathing cycle B comprises an inspiratory phase, In, and an expiratory phase, Ex.

**Figs. 4a** and **4b** illustrate volume flow to a patient (Q patient) and expiratory pressure control valve pressure level (p PEEP value) setting during pressure controlled or support ventilation. The expiratory pressure control valve is cycled between a Pressure Above PEEP, PAP, level setting during the inspiratory phase, In, and a PEEP level setting during the expiratory phase, Ex.

**Figs. 4c** and **4d** illustrate volume flow to a patient (Q patient) and patient pressure (p patient) during volume controlled or support ventilation. The expiratory pressure control valve is cycled between a closed setting during the inspiratory phase In and a PEEP level setting during the expiratory phase, Ex. During a brief phase with zero-flow to the patient at the end of the inspiratory phase, In, the patient pressure falls back to a plateau pressure, PP, level before the expiatory phase, Ex. Accordingly, these are pressure level settings of the expiratory pressure control valve 21, which may be used in some of the above discussed embodiments.

The disclosure also relates to a computer program. More specifically, to a computer program for performing a method 100 for maintaining ventilation during an oxygen flush in an anaesthetic breathing apparatus 2, wherein the computer program comprises computer readable code configured to cause one or more calculation units of one or more control units of the breathing apparatus to perform a method 100 according to any one of the aspects and/or embodiments discussed herein.

One skilled in the art will appreciate that the method 100 for maintaining ventilation during an oxygen flush in an anaesthetic breathing apparatus 2 may be implemented by programmed instructions. These programmed instructions are typically constituted by a computer program, which, when it is executed in a computer or control unit 30, see Fig. **2****,** ensures that the computer or control unit carries out the desired control, such as the method steps 102 - 122 according to the disclosure.

The computer program may be part of a computer programme product which comprises a suitable digital storage medium on which the computer program is stored, such as, for example, the computer program product 90 discussed below with reference to **Fig. 5****.**

**Fig. 5** illustrates a computer program product 90 according to embodiments. More specifically, to a computer program product 90 for performing a method 100 for maintaining ventilation during an oxygen flush in an anaesthetic breathing apparatus, wherein the computer program product 90 comprises computer readable code stored on a hardware storage device configured to cause one or more calculation units of one or more control units of the breathing apparatus to perform a method 100 according to any one of aspects and/or embodiments discussed herein. The computer readable code may be embedded in the one or more control units of the breathing apparatus and configured to operate the one or more control units to operate the breathing apparatus so as to perform the method 100 according to any one of aspects and/or embodiments discussed herein.

The computer program product may be provided, for instance, in the form of a data carrier carrying computer program code for performing at least some of the method steps 102 - 122 according to some embodiments when being loaded into the one or more calculation units 36 of a control unit 30, see **Fig. 2****.** The computer program product 90 may be, e.g., a ROM (read-only memory), a PROM (programable read-only memory), an EPROM (erasable PROM), a flash memory, an EEPROM (electrically erasable PROM), a hard disc, a CD ROM disc as disclosed in **Fig. 5****,** a memory stick, an optical storage device, a magnetic storage device or any other appropriate hardware medium such as a disk or tape that may hold machine readable data in a non-transitory manner. The computer program product 90 may furthermore be provided as computer program code on a server and may be downloaded to the control unit 30 remotely, e.g., over an Internet or an intranet connection, or via other wired or wireless communication systems.

It is to be understood that the foregoing is illustrative of various example embodiments and that the invention is defined only by the appended claims.

## Claims

1. An anaesthetic breathing apparatus (2) comprising:
a breathing circuit (3);
a fresh gas section (5) for providing fresh gas to a patient (8); and
a ventilator section (7) for providing ventilation by providing driving gas for inspiration and controlling flow of expiratory gas from the patient (8), wherein
the fresh gas section (5) and the ventilator section (7) are connected to the breathing circuit (3), wherein
the fresh gas section (5) comprises an oxygen supply (12) and at least one further gas supply (16), and the ventilator section (7) comprises an expiratory pressure control valve (21), a driving gas line (43), an inspiratory pressure control valve (44), and a rebreathing gas reservoir (20), wherein
the anaesthetic breathing apparatus (2) is configured, upon user input, to:
- start (102) a continuous flow of oxygen from the oxygen supply (12) into at least a portion of the breathing circuit (3), in order activate an oxygen flush, and
- maintain (104) the continuous flow of oxygen, **characterized in that**
the anaesthetic breathing apparatus further comprises one or more control units configured to:
- interrupt an ongoing ventilation, and
- cycle (106) the expiratory pressure control valve (21) between an upper pressure level setting to provide an inspiratory phase of a breathing cycle and a lower pressure level setting to provide an expiratory phase of the breathing cycle.

2. The anaesthetic breathing apparatus (2) according to claim 1, wherein the step of cycling (106) the expiratory pressure control valve (21) between the lower pressure level setting and the upper pressure level setting is performed at a previously set respiratory rate and a previously set ratio between the inspiratory phase and the expiratory phase.

3. The anaesthetic breathing apparatus (2) according to claim 1 or 2, wherein the lower pressure level setting is a PEEP level setting of the expiratory pressure control valve (21).

4. The anaesthetic breathing apparatus (2) according to any one of the preceding claims, wherein the upper pressure level setting is at a level below a threshold pressure level of a pressure relief valve (29) of the anaesthetic breathing apparatus (2).

5. The anaesthetic breathing apparatus (2) according to any one of the preceding claims, wherein the upper pressure level setting is at a plateau pressure level as determined at inspiratory zero-flow to a patient (8).

6. The anaesthetic breathing apparatus (2) according to any one of claims 1 - 4, wherein the upper pressure level is at a pressure above PEEP level.

7. The anaesthetic breathing apparatus (2) according to any one of the preceding claims, wherein when the step of starting (102) a continuous flow of oxygen is performed, the anaesthetic breathing apparatus (2) is configured to perform a substantially simultaneous step of:
- triggering (108) an expiratory phase with the expiratory pressure control valve (21) by performing a step of:
- setting (110) the expiratory pressure control valve (21) at the lower pressure level setting.

8. The anaesthetic breathing apparatus (2) according to any one of claims 1 - 6, wherein when the step of starting (102) a continuous flow of oxygen is performed amidst an inspiratory phase during ventilation, the anaesthetic breathing apparatus (2) is configured to continue the inspiratory phase by performing a step of:
- setting (112) the expiratory pressure control valve (21) at the higher pressure level setting for a remaining time period of the inspiratory phase, and the step of cycling (106) the expiratory pressure control valve (21) is preceded by a step of:
- providing (122) an expiratory phase supplementing the inspiratory phase to complete a breathing cycle by setting the expiratory pressure control valve (21) at the lower pressure level setting.

9. The anaesthetic breathing apparatus (2) according to any one of the preceding claims, wherein the fresh gas section (5) comprises an oxygen valve (15) for controlling flow of oxygen, and wherein the step of starting (102) a continuous flow of oxygen from the oxygen supply (12) comprises a step of:
- opening (114) at least partially the oxygen valve (15).

10. The anaesthetic breathing apparatus (2) according to any one of claims 1 - 8, wherein the anaesthetic breathing apparatus (2) comprises a dedicated oxygen flush valve (28) connected to the oxygen supply (12), and wherein the step of starting (102) a continuous flow of oxygen from the oxygen supply (12) comprises a step of:
- opening (116) at least partially the dedicated oxygen flush valve (28).

11. The anaesthetic breathing apparatus (2) according to any one of the preceding claims, wherein the anaesthetic breathing apparatus (2) is configured to precede the step of starting (102) a continuous flow of oxygen by a step of:
- ventilating (118) a patient (8) in a volume or pressure controlled mode of ventilation of the anaesthetic breathing apparatus (2), or in a volume or pressure support mode of ventilation of the anaesthetic breathing apparatus (2).

12. The anaesthetic breathing apparatus (2) according to claim 11, wherein the step of ventilating (118) a patient (8) comprises a step of:
- recirculating (120) a portion of expiratory gas from the patient (8) in the anaesthetic breathing apparatus (2).

13. A computer program that causes an anaesthetic breathing apparatus to perform a method (100) for maintaining ventilation during an oxygen flush in the anaesthetic breathing apparatus (2), wherein the computer program is embedded in a computer of the anaesthetic breathing apparatus (2) and comprises a computer readable code configured to cause one or more calculation units of one or more control units (30) of the anaesthetic breathing apparatus (2) to perform the method (100);
wherein the anaesthetic breathing apparatus (2) comprises
a breathing circuit (3), a fresh gas section (5) for providing fresh gas to a patient (8), and a ventilator section (7) for providing ventilation by providing driving gas for inspiration and controlling flow of expiratory gas from the patient (8), wherein
the fresh gas section (5) and the ventilator section (7) are connected to the breathing circuit, wherein
the fresh gas section (5) comprises an oxygen supply (12) and at least one further gas supply (16), and the ventilator section (7) comprises an expiratory pressure control valve (21), a driving gas line (43), an inspiratory pressure control valve (44), and a rebreathing gas reservoir (20),
and wherein the method (100) comprises the steps of:
- starting (102) a continuous flow of oxygen from the oxygen supply (12) into at least a portion of the breathing circuit (3) and interrupting an ongoing ventilation in order to activate the oxygen flush,
- maintaining (104) the continuous flow of oxygen, and during the step of maintaining (104) the continuous flow of oxygen performing a step of:
- cycling (106) the expiratory pressure control valve (21) between an upper pressure level setting to provide an inspiratory phase of a breathing cycle and a lower pressure level setting to provide an expiratory phase of the breathing cycle.

14. A data carrier carrying computer program code (90) that is connectable to a computer of an anaesthetic breathing apparatus (2), wherein the computer operates the anaesthetic breathing apparatus (2) so as to perform a method (100) for maintaining ventilation during an oxygen flush in the anaesthetic breathing apparatus (2), wherein the data carrier carrying computer program code (90) comprises computer readable code configured to cause one or more calculation units of one or more control units (30) of the computer of the anaesthetic breathing apparatus (2) to perform the method (100); -
wherein the anaesthetic breathing apparatus (2) comprises
a breathing circuit (3), a fresh gas section (5) for providing fresh gas to a patient (8), and a ventilator section (7) for providing ventilation by providing driving gas for inspiration and controlling flow of expiratory gas from the patient (8), wherein
the fresh gas section (5) and the ventilator section (7) are connected to the breathing circuit, wherein
the fresh gas section (5) comprises an oxygen supply (12) and at least one further gas supply (16), and the ventilator section (7) comprises an expiratory pressure control valve (21), a driving gas line (43), an inspiratory pressure control valve (44), and a rebreathing gas reservoir (20),
and wherein the method (100) comprises the steps of:
- starting (102) a continuous flow of oxygen from the oxygen supply (12) into at least a portion of the breathing circuit (3) and interrupting an ongoing ventilation in order to activate the oxygen flush,
- maintaining (104) the continuous flow of oxygen, and during the step of maintaining (104) the continuous flow of oxygen performing a step of:
- cycling (106) the expiratory pressure control valve (21) between an upper pressure level setting to provide an inspiratory phase of a breathing cycle and a lower pressure level setting to provide an expiratory phase of the breathing cycle.

## Patentansprüche

1. Narkosebeatmungsgerät (2), umfassend:
einen Beatmungskreislauf (3);
einen Frischgasabschnitt (5) zum Bereitstellen von Frischgas an einen Patienten (8); und
einen Beatmungsabschnitt (7) zum Bereitstellen von Beatmung durch Bereitstellung von Einleitgas zur Einatmung und zur Regelung von exspiratorischem Gas vom Patient (8), wobei
der Frischgasabschnitt (5) und der Beatmungsabschnitt (7) mit dem Beatmungskreislauf (3) verbunden sind, wobei
der Frischgasabschnitt (5) eine Sauerstoffzufuhr (12) und mindestens eine weitere Gaszufuhr (16) umfasst, und der Beatmungsabschnitt (7) ein exspiratorisches Druckregelventil (21), eine Einleitgasleitung (43), ein inspiratorisches Druckregelventil (44), und einen Rückatmungsgasbehälter (20) umfasst, wobei
das Narkosebeatmungsgerät (2) nach Benutzereingabe konfiguriert ist zum:
- Starten (102) eines kontinuierlichen Sauerstoffflusses von der Sauerstoffzufuhr (12) in mindestens einen Teil des Beatmungskreislaufs (3), um eine Sauerstoffspülung zu aktivieren, und
- Aufrechterhalten (104) des kontinuierlichen Sauerstoffflusses, **dadurch gekennzeichnet, dass** das Narkosebeatmungsgerät ferner eine oder mehrere Steuereinheiten umfasst, die konfiguriert sind zum:
- Unterbrechen einer fortlaufenden Beatmung, und
- zyklisches Umschalten (106) des exspiratorischen Druckregelventils (21) zwischen einer oberen Druckniveaueinstellung, um eine inspiratorische Phase eines Beatmungszyklus bereitzustellen, und einer unteren Druckniveaueinstellung, um eine exspiratorische Phase des Beatmungszyklus bereitzustellen.

2. Narkosebeatmungsgerät (2) nach Anspruch 1, wobei der Schritt des zyklischen Umschaltens (106) des exspiratorischen Druckregelventils (21) zwischen der unteren Druckniveaueinstellung und der oberen Druckniveaueinstellung bei einer zuvor eingestellten respiratorischen Rate und einem zuvor eingestellten Verhältnis zwischen der inspiratorischen Phase und der exspiratorischen Phase durchgeführt wird.

3. Narkosebeatmungsgerät (2) nach Anspruch 1 oder 2, wobei die untere Druckniveaueinstellung eine PEEP-Pegeleinstellung des exspiratorischen Druckregelventils (21) ist.

4. Narkosebeatmungsgerät (2) nach einem der vorstehenden Ansprüche, wobei die untere Druckniveaueinstellung sich auf dem Niveau unterhalb eines Schwellendruckniveaus eines Druckentlastungsventils (29) des Narkosebeatmungsgeräts (2) befindet.

5. Narkosebeatmungsgerät (2) nach einem der vorstehenden Ansprüche, wobei die obere Druckniveaueinstellung sich auf dem Plateaudruckniveau befindet, das bei einem inspiratorischen Nullfluss zu einem Patienten (8) bestimmt wird.

6. Narkosebeatmungsgerät (2) nach einem der Ansprüche 1 bis 4, wobei das obere Druckniveau bei einem Druck über PEEP-Niveau liegt.

7. Narkosebeatmungsgerät (2) nach einem der vorhergehenden Ansprüche, wobei, wenn der Schritt des Startens (102) eines kontinuierlichen Sauerstoffflusses durchgeführt wird, das Narkosebeatmungsgerät (2) konfiguriert ist zum Durchführen eines im Wesentlichen gleichzeitigen Schritts des:
- Auslösens (108) einer exspiratorischen Phase mit dem exspiratorischen Druckregelventil (21) durch Durchführen eines Schritts des:
- Einstellens (110) des exspiratorischen Druckregelventils (21) auf die untere Druckniveaueinstellung.

8. Narkosebeatmungsgerät (2) nach einem der Ansprüche 1 bis 6, wobei, wenn der Schritt des Startens (102) eines kontinuierlichen Sauerstoffflusses inmitten einer inspiratorischen Phase während der Beatmung durchgeführt wird, das Narkosebeatmungsgerät (2) konfiguriert ist zum Fortsetzen der inspiratorischen Phase durch Durchführen eines Schritts des:
- Einstellens (112) des exspiratorischen Druckregelventils (21) auf die höhere Druckniveaueinstellung für einen verbleibenden Zeitraum der inspiratorischen Phase, und wobei dem Schritt des zyklischen Umschaltens (106) des exspiratorischen Druckregelventils (21) ein Schritt vorausgeht des:
- Bereitstellens (122) einer exspiratorischen Phase, die die inspiratorische Phase ergänzt, um einen Beatmungszyklus abzuschließen, indem das exspiratorische Druckregelventil (21) auf die untere Druckniveaueinstellung eingestellt wird.

9. Narkosebeatmungsgerät (2) nach einem der vorstehenden Ansprüche, wobei der Frischgasabschnitt (5) ein Sauerstoffventil (15) zum Regeln des Sauerstoffflusses umfasst, und wobei der Schritt des Startens (102) eines kontinuierlichen Sauerstoffflusses von der Sauerstoffzufuhr (12) einen Schritt umfasst des:
- mindestens teilweises Öffnens (114) des Sauerstoffventils (15).

10. Narkosebeatmungsgerät (2) nach einem der Ansprüche 1 bis 8, wobei das Narkosebeatmungsgerät (2) ein dediziertes Sauerstoffspülventil (28) umfasst, das mit der Sauerstoffzufuhr (12) verbunden ist, und wobei der Schritt des Startens (102) eines kontinuierlichen Sauerstoffflusses von der Sauerstoffzufuhr (12) einen Schritt umfasst des:
- mindestens teilweises Öffnens (116) des dedizierten Sauerstoffspülventils (28).

11. Narkosebeatmungsgerät (2) nach einem der vorstehenden Ansprüche, wobei das Narkosebeatmungsgerät (2) so konfiguriert ist, dass dem Schritt des Startens (102) eines kontinuierlichen Sauerstoffflusses ein Schritt vorausgeht des:
- Beatmens (118) eines Patienten (8) in einem volumen- oder druckgesteuerten Beatmungsmodus des Narkosebeatmungsgeräts (2) oder in einem volumen- oder druckunterstützenden Beatmungsmodus des Narkosebeatmungsgeräts (2).

12. Narkosebeatmungsgerät (2) nach Anspruch 11, wobei der Schritt des Beatmens (118) eines Patienten (8) einen Schritt umfasst des:
- Rezirkulierens (120) eines Teils von exspiratorischem Gas von dem Patienten (8) in dem Narkosebeatmungsgerät (2).

13. Computerprogramm, das ein Narkosebeatmungsgerät veranlasst, ein Verfahren (100) zum Aufrechterhalten von Beatmung während einer Sauerstoffspülung in dem Narkosebeatmungsgerät (2) durchzuführen, wobei das Computerprogramm in einen Computer des Narkosebeatmungsgeräts (2) eingebettet ist und einen computerlesbaren Code umfasst, der konfiguriert ist, eine oder mehrere Berechnungseinheiten einer oder mehrerer Steuereinheiten (30) des Narkosebeatmungsgeräts (2) zu veranlassen, das Verfahren (100) durchzuführen;
wobei das Narkosebeatmungsgerät (2) umfasst
einen Beatmungskreislauf (3), einen Frischgasabschnitt (5) zum Bereitstellen von Frischgas an einen Patienten (8) und einen Beatmungsabschnitt (7) zum Bereitstellen von Beatmung durch Bereitstellen von Einleitgas für die Einatmung und Regeln des Flusses von exspiratorischem Gas von dem Patienten (8), wobei
der Frischgasabschnitt (5) und der Beatmungsabschnitt (7) mit dem Beatmungskreislauf verbunden sind, wobei
der Frischgasabschnitt (5) eine Sauerstoffzufuhr (12) und mindestens eine weitere Gaszufuhr (16) umfasst und der Beatmungsabschnitt (7) ein exspiratorisches Druckregelventil (21), eine Einleitgasleitung (43), ein inspiratorisches Druckregelventil (44) und einen Rückatmungsgasbehälter (20) umfasst,
und wobei das Verfahren (100) die Schritte umfasst:
- Starten (102) eines kontinuierlichen Sauerstoffflusses von der Sauerstoffzufuhr (12) in mindestens einen Teil des Beatmungskreislaufs (3) und Unterbrechen einer fortlaufenden Beatmung, um die Sauerstoffspülung zu aktivieren,
- Aufrechterhalten (104) des kontinuierlichen Sauerstoffflusses, und während des Schritts des Aufrechterhaltens (104) des kontinuierlichen Sauerstoffflusses, Durchführen eines Schritts:
- zyklisches Umschalten (106) des exspiratorischen Druckregelventils (21) zwischen einer oberen Druckniveaueinstellung, um eine inspiratorische Phase eines Beatmungszyklus bereitzustellen, und einer unteren Druckniveaueinstellung, um eine exspiratorische Phase des Beatmungszyklus bereitzustellen.

14. Datenträger, der Computerprogrammcode (90) trägt, der mit einem Computer eines Narkosebeatmungsgeräts (2) verbunden werden kann, wobei der Computer das Narkosebeatmungsgerät (2) derart betreibt, dass ein Verfahren (100) zum Aufrechterhalten einer Beatmung während einer Sauerstoffspülung in dem Narkosebeatmungsgerät (2) durchgeführt wird, wobei der Datenträger, der Computerprogrammcode (90) trägt, computerlesbaren Code umfasst, der konfiguriert ist, eine oder mehrere Berechnungseinheiten einer oder mehrerer Steuereinheiten (30) des Computers des Narkosebeatmungsgerät (2) zu veranlassen, das Verfahren (100) durchzuführen; -
wobei das Narkosebeatmungsgerät (2) umfasst
einen Beatmungskreislauf (3), einen Frischgasabschnitt (5) zum Bereitstellen von Frischgas an einen Patienten (8) und einen Beatmungsabschnitt (7) zum Bereitstellen von Beatmung durch Bereitstellen von Einleitgas für die Einatmung und Regeln des Flusses von exspiratorischem Gas von dem Patienten (8), wobei
der Frischgasabschnitt (5) und der Beatmungsabschnitt (7) mit dem Beatmungskreislauf verbunden sind, wobei
der Frischgasabschnitt (5) eine Sauerstoffzufuhr (12) und mindestens eine weitere Gaszufuhr (16) umfasst und der Beatmungsabschnitt (7) ein exspiratorisches Druckregelventil (21), eine Einleitgasleitung (43), ein inspiratorisches Druckregelventil (44) und einen Rückatmungsgasbehälter (20) umfasst,
und wobei das Verfahren (100) die Schritte umfasst:
- Starten (102) eines kontinuierlichen Sauerstoffflusses von der Sauerstoffzufuhr (12) in mindestens einen Teil des Beatmungskreislaufs (3) und Unterbrechen einer fortlaufenden Beatmung, um die Sauerstoffspülung zu aktivieren,
- Aufrechterhalten (104) des kontinuierlichen Sauerstoffflusses, und während des Schritts des Aufrechterhaltens (104) des kontinuierlichen Sauerstoffflusses, Durchführen eines Schritts:
- zyklisches Umschalten (106) des exspiratorischen Druckregelventils (21) zwischen einer oberen Druckniveaueinstellung, um eine inspiratorische Phase eines Beatmungszyklus bereitzustellen, und einer unteren Druckniveaueinstellung, um eine exspiratorische Phase des Beatmungszyklus bereitzustellen.

## Revendications

1. Appareil respiratoire d'anesthésie (2) comprenant :
un circuit respiratoire (3) ;
une section de gaz frais (5) pour fournir du gaz frais à un patient (8) ; et
une section de ventilateur (7) pour fournir une ventilation en fournissant un gaz d'entraînement pour l'inspiration et en commandant le débit de gaz expiratoire provenant du patient (8), dans lequel
la section de gaz frais (5) et la section de ventilateur (7) sont reliées au circuit respiratoire (3), dans lequel
la section de gaz frais (5) comprend une alimentation en oxygène (12) et au moins une autre alimentation en gaz (16), et la section de ventilation (7) comprend une soupape de commande de pression expiratoire (21), une conduite de gaz d'entraînement (43), une soupape de commande de pression inspiratoire (44) et un réservoir de gaz respiratoire (20), dans lequel
l'appareil respiratoire d'anesthésie (2) est configuré, après une intervention de l'utilisateur, pour :
- démarrer (102) un flux continu d'oxygène à partir de l'alimentation en oxygène (12) dans au moins une partie du circuit respiratoire (3), afin d'activer un rinçage à l'oxygène, et
- maintenir (104) le flux continu d'oxygène, **caractérisé en ce que** l'appareil respiratoire d'anesthésie comprend en outre une ou plusieurs unités de commande configurées pour :
- interrompre une ventilation en cours, et
- cycler (106) la soupape de commande de pression expiratoire (21) entre un réglage de niveau de pression supérieur pour fournir une phase inspiratoire d'un cycle respiratoire et un réglage de niveau de pression inférieur pour fournir une phase expiratoire du cycle respiratoire.

2. Appareil respiratoire d'anesthésie (2) selon la revendication 1, dans lequel l'étape de cyclage (106) de la soupape de commande de pression expiratoire (21) entre le réglage de niveau de pression inférieur et le réglage de niveau de pression supérieur est effectuée à une fréquence respiratoire préalablement réglée et à un rapport préalablement réglé entre la phase inspiratoire et la phase expiratoire.

3. Appareil respiratoire d'anesthésie (2) selon la revendication 1 ou 2, dans lequel le réglage de niveau de pression inférieur est un réglage de niveau PEEP de la soupape de commande de pression expiratoire (21).

4. Appareil respiratoire d'anesthésie (2) selon l'une quelconque des revendications précédentes, dans lequel le réglage de niveau de pression supérieur est à un niveau inférieur à un niveau de pression seuil d'une soupape de sûreté de pression (29) de l'appareil respiratoire d'anesthésie (2).

5. Appareil respiratoire d'anesthésie (2) selon l'une quelconque des revendications précédentes, dans lequel le réglage de niveau de pression supérieur est à un niveau de pression de plateau tel que déterminé à un flux inspiratoire nul vers un patient (8).

6. Appareil respiratoire d'anesthésie (2) selon l'une quelconque des revendications 1 à 4, dans lequel le réglage de niveau de pression supérieur est à une pression supérieure au niveau PEP.

7. Appareil respiratoire d'anesthésie (2) selon l'une quelconque des revendications précédentes, dans lequel lorsque l'étape de démarrage (102) d'un flux continu d'oxygène est effectuée, l'appareil respiratoire d'anesthésie (2) est configuré pour effectuer une étape essentiellement simultanée consistant à :
- déclencher (108) une phase expiratoire avec la soupape de commande de pression expiratoire (21) en effectuant une étape consistant à :
- régler (110) la soupape de commande de pression expiratoire (21) au réglage de niveau de pression inférieur.

8. Appareil respiratoire d'anesthésie (2) selon l'une quelconque des revendications 1 à 6, dans lequel lorsque l'étape de démarrage (102) d'un flux continu d'oxygène est effectuée au milieu d'une phase inspiratoire pendant la ventilation, l'appareil respiratoire d'anesthésie (2) est configuré pour poursuivre la phase inspiratoire en effectuant une étape consistant à :
- régler (112) la soupape de commande de pression expiratoire (21) au réglage de niveau de pression supérieur pendant une période de temps restante de la phase inspiratoire, et l'étape de cyclage (106) de la soupape de commande de pression expiratoire (21) est précédée d'une étape consistant à :
- fournir (122) une phase expiratoire complétant la phase inspiratoire pour terminer un cycle respiratoire en réglant la soupape de commande de pression expiratoire (21) au réglage de niveau de pression inférieur.

9. Appareil respiratoire d'anesthésie (2) selon l'une quelconque des revendications précédentes, dans lequel la section de gaz frais (5) comprend une soupape à oxygène (15) pour contrôler le flux d'oxygène, et dans lequel l'étape de démarrage (102) d'un flux continu d'oxygène depuis l'alimentation en oxygène (12) comprend une étape consistant à :
- ouvrir (114) au moins partiellement la soupape à oxygène (15).

10. Appareil respiratoire d'anesthésie (2) selon l'une quelconque des revendications 1 à 8, dans lequel l'appareil respiratoire d'anesthésie (2) comprend une soupape de rinçage à l'oxygène dédiée (28) reliée à l'alimentation en oxygène (12), et dans lequel l'étape de démarrage (102) d'un flux continu d'oxygène provenant de l'alimentation en oxygène (12) comprend une étape consistant à
- ouvrir (116) au moins partiellement la soupape de rinçage à l'oxygène dédiée (28).

11. Appareil respiratoire d'anesthésie (2) selon l'une quelconque des revendications précédentes, dans lequel l'appareil respiratoire d'anesthésie (2) est configuré pour précéder l'étape de démarrage (102) d'un flux continu d'oxygène par une étape de :
- la ventilation (118) d'un patient (8) dans un mode commandé de ventilation en volume ou en pression de l'appareil respiratoire d'anesthésie (2), ou dans un mode de support de ventilation en volume ou en pression de l'appareil respiratoire d'anesthésie (2).

12. Appareil respiratoire d'anesthésie (2) selon la revendication 11, dans lequel l'étape de ventilation (118) d'un patient (8) comprend une étape consistant à :
- recirculer (120) une partie du gaz expiratoire provenant du patient (8) dans l'appareil respiratoire d'anesthésie (2).

13. Programme informatique qui amène un appareil respiratoire d'anesthésie à réaliser un procédé (100) pour maintenir une ventilation pendant un rinçage à l'oxygène dans l'appareil respiratoire d'anesthésie (2), le programme informatique étant intégré dans un ordinateur de l'appareil respiratoire d'anesthésie (2) et comprenant un code lisible par ordinateur configuré pour amener une ou plusieurs unités de calcul d'une ou de plusieurs unités de commande (30) de l'appareil respiratoire d'anesthésie (2) à réaliser le procédé (100) ;
l'appareil respiratoire d'anesthésie (2) comprenant
un circuit respiratoire (3), une section de gaz frais (5) pour fournir du gaz frais à un patient (8), et une section de ventilateur (7) pour fournir une ventilation en fournissant un gaz d'entraînement pour l'inspiration et en commandant le flux de gaz expiratoire provenant du patient (8), dans lequel
la section de gaz frais (5) et la section de ventilateur (7) sont reliées au circuit respiratoire, dans lequel
la section de gaz frais (5) comprend une alimentation en oxygène (12) et au moins une autre alimentation en gaz (16), et la section de ventilateur (7) comprend une soupape de commande de pression expiratoire (21), une conduite de gaz d'entraînement (43), une soupape de commande de pression inspiratoire (44) et un réservoir de gaz respiratoire (20),
dans lequel le procédé (100) comprend les étapes consistant à :
- démarrer (102) un flux continu d'oxygène à partir de l'alimentation en oxygène (12) dans au moins une partie du circuit respiratoire (3) et interrompre une ventilation en cours, afin d'activer le rinçage à l'oxygène,
- maintenir (104) le flux continu d'oxygène, et au cours de l'étape de maintien (104) du flux continu d'oxygène, réaliser une étape consistant à :
- cycler (106) la soupape de commande de pression expiratoire (21) entre un réglage de niveau de pression supérieur pour fournir une phase inspiratoire d'un cycle respiratoire et un réglage de niveau de pression inférieur pour fournir une phase expiratoire du cycle respiratoire.

14. Support de données transportant un code de programme informatique (90) qui peut être connecté à un ordinateur d'un appareil respiratoire d'anesthésie (2), l'ordinateur actionnant l'appareil respiratoire d'anesthésie (2) de façon à effectuer un procédé (100) pour maintenir une ventilation pendant un rinçage à l'oxygène dans l'appareil respiratoire d'anesthésie (2), le support de données transportant un code de programme informatique (90) comprenant un code lisible par ordinateur configuré pour amener une ou plusieurs unités de calcul d'une ou de plusieurs unités de commande (30) de l'ordinateur de l'appareil respiratoire d'anesthésie (2) à réaliser le procédé (100) ;
l'appareil respiratoire d'anesthésie (2) comprenant
un circuit respiratoire (3), une section de gaz frais (5) pour fournir du gaz frais à un patient (8), et une section de ventilateur (7) pour fournir une ventilation en fournissant un gaz d'entraînement pour l'inspiration et en commandant le flux de gaz expiratoire provenant du patient (8), dans lequel
la section de gaz frais (5) et la section de ventilateur (7) sont reliées au circuit respiratoire, dans lequel
la section de gaz frais (5) comprend une alimentation en oxygène (12) et au moins une autre alimentation en gaz (16), et la section de ventilation (7) comprend une soupape de commande de pression expiratoire (21), une conduite de gaz d'entraînement (43), une soupape de commande de pression inspiratoire (44) et un réservoir de gaz respiratoire (20),
dans lequel le procédé (100) comprend les étapes consistant à :
- démarrer (102) un flux continu d'oxygène à partir de l'alimentation en oxygène (12) dans au moins une partie du circuit respiratoire (3) et interrompre une ventilation en cours, afin d'activer le rinçage à l'oxygène,
- maintenir (104) le flux continu d'oxygène, et au cours de l'étape de maintien (104) du flux continu d'oxygène, réaliser une étape consistant à :
- cycler (106) la soupape de commande de pression expiratoire (21) entre un réglage de niveau de pression supérieur pour fournir une phase inspiratoire d'un cycle respiratoire et un réglage de niveau de pression inférieur pour fournir une phase expiratoire du cycle respiratoire.
